Europäisches Patentamt

**European Patent Office**

**Office européen des brevets**

(11) Numero de publication: **0 139 546**
**A2**

(12) # DEMANDE DE BREVET EUROPEEN

(21) Numéro de dépôt: **84401582.6**

(22) Date de dépôt: **27.07.84**

(51) Int. Cl.⁴: **C 12 P 7/28**
C 12 P 7/04, C 12 P 7/06
//(C12P7/28, C12R1:145),
(C12P7/04, C12R1:145),
(C12P7/06, C12R1:145)

(30) Priorité: **29.07.83 FR 8312522**

(43) Date de publication de la demande:
**02.05.85 Bulletin 85/18**

(84) Etats contractants désignés:
**AT BE CH DE FR GB IT LI LU NL SE**

(71) Demandeur: **INSTITUT PASTEUR**
**25-28, rue du Docteur Roux**
**F-75724 Paris Cedex 15(FR)**

(72) Inventeur: **Zeikus, Joseph Gregory**
**3229 Knollwood Way**
**Madison Wisconsin 53703(US)**

(72) Inventeur: **Longin, Robert**
**104 Rue Sadi Carnot**
**F-92170 Vanves(FR)**

(74) Mandataire: **Ores, Irène et al,**
**CABINET ORES 6, Avenue de Messine**
**F-75008 Paris(FR)**

(54) **Procédé de production d'alcools par des fermentations microbiennes thermophiles, à de hautes concentrations de solvants organiques.**

(57) La présente invention est relative à un procédé de production d'alcools par des fermentations microbiennes.

Ce procédé se caractérise par la bioconversion d'au moins un substrat constitué par des composés organiques oxygénés, à l'aide d'au moins un microorganisme thermophile anaérobie résistant aux alcools obtenus, à température élevée, de préférence comprise entre 40 et 75°C, et plus particulièrement à la température de croissance du ou des microorganisme(s) thermophile(s) anaérobie(s), en présence de concentrations égales ou supérieures à 1 % dudit produit final.

Application : production d'alcools par fermentation.

EP 0 139 546 A2

./...

FIG. 1

-1-

La présente invention est relative à un procédé de production d'alcools par des fermentations microbiennes thermophiles, à de hautes concentrations de solvants organiques.

La possibilité de convertir des ressources renouvelables, telles que la biomasse, ou les déchets, en produits chimiques présentant un intérêt industriel et en combustibles, par fermentation, constitue un objectif d'un intérêt économique considérable. En effet, on sait que les fermentations anaérobies tranforment la matière organique en l'absence d'oxygène, pour donner un certain nombre de composés organiques plus ou moins réduits tels que l'éthanol, le méthane, l'acide acétique, le butanol, par exemple.

Les premières applications de ces fermentations remontent à Louis PASTEUR /Bull. Soc. Chim. Paris, 1862 (1):53/ qui a obtenu du butanol par fermentation bactérienne et à Ch. WEIZMANN qui, en 1912, a converti de l'amidon en acétone, butanol et éthanol par fermentation à l'aide d'un microorganisme dénommé "Clostridium acétobutylicum souche Weizmann" ; le butanol et l'acétone ainsi obtenus par fermentations bactériennes étaient utilisés comme matières premières, le premier pour la production de butadiène servant à la synthèse du caoutchouc synthétique et le second pour la fabrication d'explosifs. Toutefois, les procédés par fermentation ont été abandonnés après la seconde guerre mondiale au profit de l'utilisation de matières premières dérivées du pétrole, pour la synthèse chimique du butanol et de l'acétone, dont le bilan économique était plus favorable que celui des procédés par fermentation.

L'augmentation considérable du prix du pétrole et la nécessité pour les fabricants de produits chimiques et pour les Etats de réduire leur dépendance vis-à-vis des pays producteurs de pétrole ont entraîné un regain d'intérêt pour les fermentations microbiennes capables de produire des combustibles et des produits chimiques à partir de substrats renouvelables peu coûteux tels que la biomasse.

-2-

Toutefois, pour que les fermentations microbiennes soient intéressantes économiquement pour la production de produits chimiques industriels, il est indispensable que non seulement la biomasse utilisée comme substrat soit peu coûteuse, mais qu'en outre ces procédés soient faibles consommateurs d'énergie, qu'ils soient capables de fournir les produits recherchés par conversion directe, et que les rendements en ces produits soient élevés.

Les bactéries thermophiles constituent une catégorie de microorganismes propres à répondre aux conditions énoncées ci-dessus ; en effet, les bactéries thermophiles croissent à des températures égales ou supérieures à 60°C, présentent des vitesses métaboliques élevées, contiennent des enzymes physiquement stables (c'est-à-dire dont la résistance thermique est élevée) et chimiquement stables et procurent un rendement en produit final particulièrement élevé. Les bactéries thermophiles anaérobies telles que <u>Clostridium thermoaceticum</u>, qui est une bactérie acétogène, <u>Clostridium thermocellum</u> qui est une bactérie cellulolytique, <u>Clostridium thermohydrosulfuricum</u> qui est une bactérie solventogène, <u>Méthanobacterium thermoautotrophicum</u>, qui est une bactérie méthanogène, par exemple, qui sont présentes dans des environnements auto-caloriques tels que le sol, ou volcaniques, permettent d'obtenir des produits de fermentation utilisables directement sur le plan industriel tels que l'acide acétique, l'éthanol, le méthane, ou utilisables comme matière première pour la production d'autres produits chimiques, comme c'est le cas, par exemple, de l'éthanol qui constitue une matière première de choix pour la production d'éthylène ou de butadiène. L'isopropanol peut être produit à partir de l'acétone obtenue par fermentation acétonobutylique. Il peut servir, par exemple, comme co-solvant à des mélanges méthanol-essence ou comme produit chimique de base. L'on sait également que si Clostridium propionicum est apte à produire de l'acide propionique, il peut, en modifiant chimiquement les paramètres de fermentation, produire de l'acide acrylique. L'on sait, en

0139546

-3-

outre, que Clostridium thermocellum et Clostridium thermohydrosulfuricum produisent de l'éthanol avec des rendements
élevés, par fermentation de saccharides, et que les rendements les plus élevés en éthanol sont obtenus par fermentation
à l'aide de souches spécifiques de ces bactéries, à savoir
les souches de C. thermocellum LQRI et de C. thermohydrosulfuricum 39E, à des températures de l'ordre de 60°C, de
substrats constitués par de la cellulose pour la première
souche et des hexoses, des pentoses ou de l'amidon pour la
seconde. Néanmoins, l'utilisation de la technologie de la
fermentation de la biomasse se heurte au problème posé par la
récupération du produit final, et en particulier de l'éthanol, produit par biofermentation et se heurte également à la
faible tolérance d'une souche telle que C. thermohydrosulfuricum à l'égard de concentrations élevées en produit final volatil tel que l'éthanol. De ce fait, en dépit du développement de techniques d'optimisation des rendements en
éthanol obtenus avec cette souche, par exemple par l'introduction de donneurs ($H_2$ par exemple) ou d'accepteurs (par
exemple l'acétone) d'électrons dans le milieu de fermentation, les technologies de fermentation de la biomasse n'ont
pas encore atteint le stade industriel.

Il a été proposé récemment d'améliorer le rendement
d'éthanol obtenu par fermentation de la cellulose, en utilisant soit une co-culture de deux microorganismes, à savoir
Thermoanaerobacter ethanolicus et Clostridium thermocellum
(cf. le Brevet américain 4 292 406) pour obtenir des quantités d'éthanol récupérables par distillation, soit une co-
culture de C. thermocellum et de C. thermohydrosulfuricum
(Appl. Environ. Microbiol. vol. 41, 1981, n° 6, p. 1337-1343,
Ng, Ben-Bassat et Zeikus). Toutefois, ce système ne permet
pas d'obtenir des concentrations d'éthanol supérieures à 1 %,
et il a par ailleurs été démontré (Appl. Environ. Microbiol.,
Vol. 40 : 571-577, Herrero and Gomez) que la présence de concentrations d'éthanol inférieures à 1 % inhibe la croissance
de C. thermocellum.

La présente invention s'est en conséquence donné pour but de pourvoir à un procédé de production d'alcools par fermentation d'une biomasse renouvelable, à l'aide d'au moins un microorganisme thermophile anaérobie spécifique, qui répond mieux aux nécessités de la pratique que les procédés visant au même but antérieurement connus, en ce qu'il permet la production desdits alcools à des concentrations égales ou supérieures à 1 %, sans provoquer d'inhibition de la croissance du microorganisme mis en oeuvre, en ce que ladite production est réalisée aussi bien en cultures continues qu'en cultures discontinues du microorganisme et en ce qu'il permet la production d'alcools en présence de concentrations élevées de solvants organiques.

La présente invention a pour objet un procédé de production d'alcools, par bioconversion d'au moins un substrat constitué par des composés organiques oxygénés, à l'aide d'au moins un microorganisme thermophile anaérobie résistant aux alcools obtenus, à température élevée, de préférence comprise entre 40 et 75°C, et plus particulièrement à la température de croissance du ou des microorganisme(s) thermophile(s) anaérobie(s), en présence de concentrations égales ou supérieures à 1 % dudit produit final.

Selon un mode de réalisation avantageux du procédé conforme à la présente invention, ledit substrat est soumis à l'action de bioconversion d'au moins un microorganisme thermophile anaérobie, en présence d'un solvant organique liquide de préférence constitué par un alcool aliphatique inférieur ou une cétone, qui stimule la croissance et/ou la production de l'alcool obtenu en tant que produite final.

Selon un autre mode de réalisation avantageux du procédé conforme à l'invention, ledit substrat est associé à un co-substrat constitué par un solvant pris dans le groupe des cétones et des aldéhydes.

Selon une disposition avantageuse de ce mode de réalisation, le substrat et le co-substrat sont associés à un solvant organique liquide, de préférence constitué par un

0139546

-5-

alcool aliphatique inférieur ou une cétone.

Selon encore un autre mode de réalisation avantageux du procédé conforme à l'invention, ledit substrat est constitué par au moins un saccharide pris dans le groupe qui comprend les pentoses, les hexoses, et autres glucides, qui est soumis à l'action de bioconversion d'au moins un microorganisme thermophile anaérobie résistant au produit organique réduit obtenu, pour fournir un alcool aliphatique inférieur tel que l'éthanol, à une concentration égale ou supérieure à 1 % et pouvant atteindre au moins 5 % (v/v) dans le système de bioconversion.

Selon une disposition avantageuse de ce mode de réalisation, le système de bioconversion comprend en outre un alcool aliphatique inférieur ou une cétone qui joue le rôle de stimulant de la croissance du microorganisme, et/ou de la production de l'alcool aliphatique inférieur recherché en tant que produit final.

Selon une modalité préférée de cette disposition, l'alcool aliphatique inférieur ou la cétone qui joue le rôle de stimulant de la croissance du microorganisme, est pris dans le groupe qui comprend le méthanol, l'éthanol, l'isopropanol, le butanol et l'acétone.

Conformément à l'invention, l'alcool qui joue le rôle de stimulant est présent dans le système de bioconversion à une concentration comprise entre 0,1 et 8,2 % en volume.

Selon un mode de réalisation avantageux de l'invention, le système de bioconversion comprend un substrat constitué par au moins un saccharide, un microorganisme thermophile anaérobie et un co-substrat pris dans le groupe qui comprend les aldéhydes et les cétones, et qui est avantageusement constitué par de l'acétone.

Selon une disposition avantageuse de ce mode de réalisation, le système de bioconversion comprend en outre un solvant organique liquide avantageusement constitué par un alcool aliphatique inférieur, pris, de préférence, dans le groupe qui comprend le méthanol et l'éthanol, le produit final obtenu par bioconversion étant essentiellement de l'isopropanol.

Conformément à ce mode de réalisation de l'invention, le co-substrat tel que l'acétone est présent à une concentration de 0,1 à 6 % en volume environ dans le système de bioconversion.

Egalement conformément à l'invention, lorsque le substrat constitué par un saccharide et le co-substrat avantageusement constitué par de l'acétone sont associés à un solvant organique liquide constitué par un alcool aliphatique inférieur ou par une cétone, l'alcool aliphatique inférieur ou la cétone est présent à une concentration comprise entre 0,1 et 8,2 % en volume.

Conformément à l'invention, le microorganisme thermophile anaérobie mis en oeuvre dans le système de bioconversion est de préférence constitué par une souche mutante de bactéries termophiles anaérobies telles que Clostridium thermosaccharolyticum ou Clostridium thermohydrosulfuricum. La souche mutante utilisée préférentiellement selon la présente invention est la souche dénommée 39EA, souche mutante de Clostridium thermohydrosulfuricum. Cette souche a été déposée dans la Collection Nationale de Cultures de Microorganismes tenue par l'Institut Pasteur sous le n°I-226 en date du 16 JUIN 1983, laquelle souche a été obtenue après transferts successifs de la souche sauvage de C.thermohydrosulfuricum 39E dans des milieux de culture contenant des concentrations croissantes d'éthanol, et isolément sur milieu solide contenant 6% d'éthanol.

Les caractéristiques comparées de croissance de la souche mutante 39EA et de la souche sauvage 39E sont reproduites dans le tableau I ci-dessous, qui fournit les données obtenues respectivement en ce qui concerne la croissance de la souche 39E et de la souche mutante 39EA sur un milieu CM3-3 contenant 5 g/litre de xylose et 5 g/litre d'extrait de levure, en l'absence de solvants, par incubation pendant 24 heures à 62°C, sans agitation.

TABLEAU I

Comparaison de la croissance des souches 39E et 39EA en l'absence de solvants

| Souche | Croissance DO à 540 nm | pH | Produits finaux (mM) | | |
|---|---|---|---|---|---|
| | | | Ethanol | Lactate | Acétate |
| 39E | 1,05 | 6,5 | 41 | 18 | 7 |
| 39EA | 1,05 | 6,4 | 17 | 22 | $< 1,0$ |

Le tableau II qui va suivre montre l'action de fermentation exercée par la souche mutante 39EA sur différents saccharides, sur milieu CM3-3 contenant 5 g/litre de substrat (saccharide), 5 % d'éthanol (v/v) et 5 g/litre d'extrait de levure, par incubation pendant 24 heures à 62°C, sans agitation.

## TABLEAU II

Fermentation de saccharides par la souche mutante 39EA
en présence de 5 % d'éthanol (v/v)

| Substrat | Croissance (DO à 540 nm) | pH final |
|---|---|---|
| Sans substrat | 0,1 | 7,5 |
| Glucose | 1,2 | 6,3 |
| Cellobiose | 1,0 | 6,4 |
| Fructose | 0,9 | 6,4 |
| Xylose | 1,0 | 6,5 |
| Lactose | 1,0 | 6,5 |

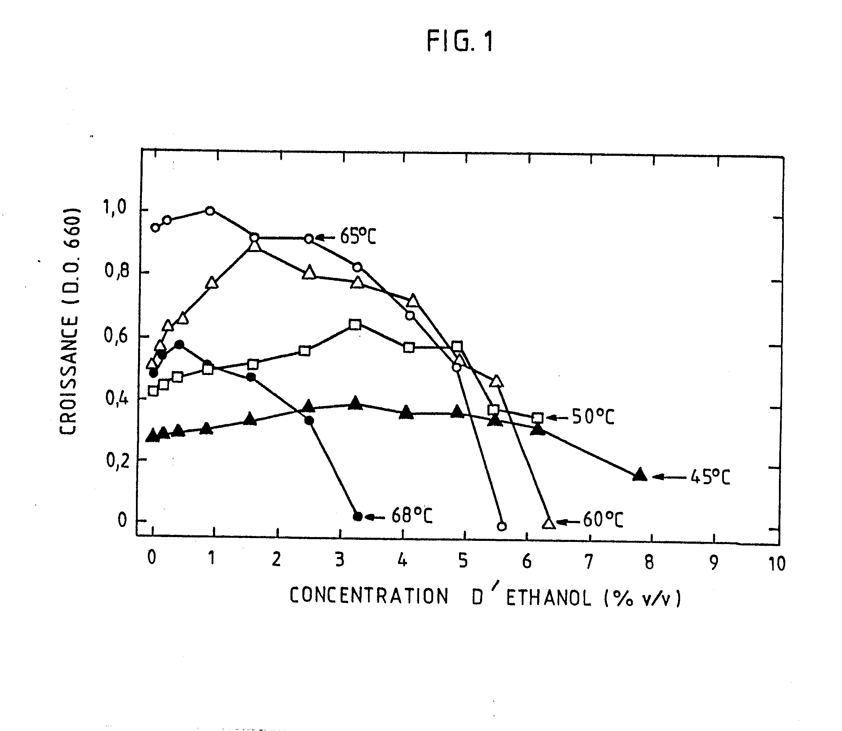

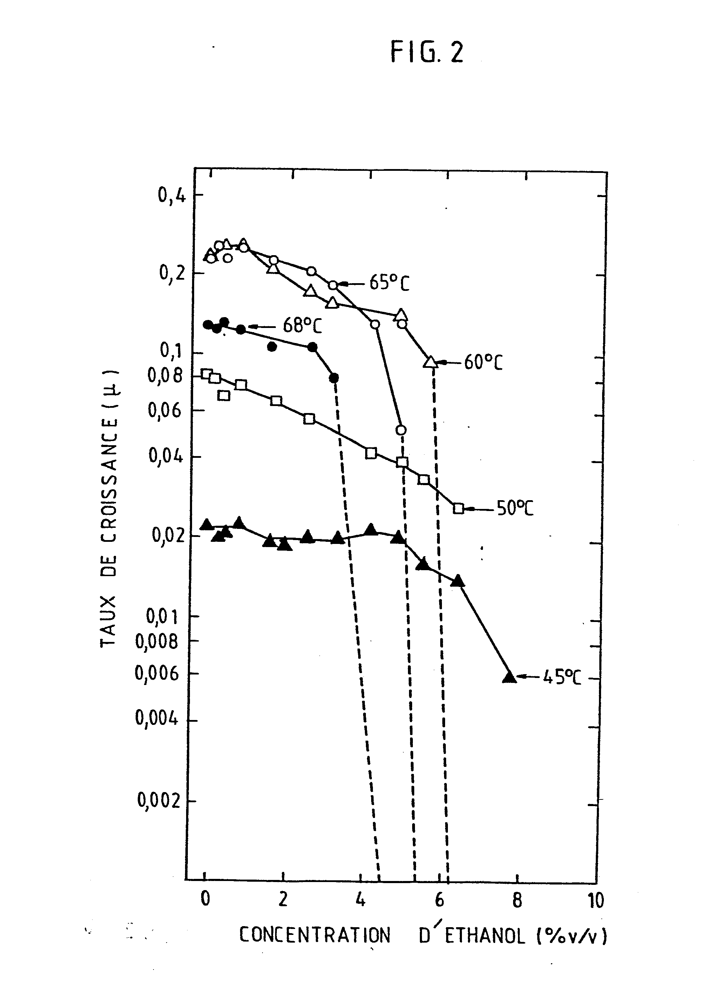

Les caractéristiques de croissance de la souche mutante 39EA sont, en outre, représentées dans les figures 1 à 3 annexées, dans lesquelles :

- la figure 1 représente par des courbes, l'influence de la concentration d'éthanol dans le milieu de bioconversion, sur la croissance de la souche 39EA, en fonction de la température,

- la figure 2 représente par des courbes, la relation entre le taux de croissance de la souche 39EA, la température et la concentration d'éthanol dans le milieu de bioconversion, et

- la figure 3 représente par des courbes, l'effet de la concentration d'éthanol dans le milieu de bioconversion, sur le rapport du taux de croissance en présence d'éthanol/ taux de croissance en l'absence d'éthanol.

Cet effet est caractérisé par le rapport du taux de croissance de l'essai en présence d'éthanol indiqué par :

(μtest ⎣éthanol⎦) sur le taux de croissance en l'absence d'éthanol, pour la souche 39EA, en fonction de la température. Cet effet est aussi montré pour la souche 39E à 65°C.

Selon un autre mode de réalisation avantageux du procédé conforme à la présente invention, la souche mutante 39EA est associée à des microorganismes ou des enzymes aptes à dégrader d'autres saccharides que les pentoses, les hexoses, et autres glucides solubles, et notamment à des microorganismes ou des enzymes aptes à dégrader la cellulose et l'hémicellulose en sucres solubles (par exemple cellobiose, glucose, xylobiose et xylose).

Selon une disposition avantageuse de ce mode de réalisation, le substrat est constitué par de la cellulose ou de l'hémicellulose.

Selon une autre disposition avantageuse de ce mode de réalisation, les microorganismes aptes à dégrader la cellulose et l'hémicellulose en hexoses, pentoses et autres glucides solubles, sont des microorganismes exerçant une activité cellulolytique tels que, notamment, Clostridium thermocel-lum souche LQRI et les enzymes cellulolytiques sont de préférence des complexes cellulasiques ou hémicellulasiques.

Conformément à l'invention, le système de bioconversion constitué par un substrat du type de la cellulose, par au moins un microorganisme ou une enzyme apte à dégrader la cellulose et les hémicelluloses en hexoses, pentoses et autres glucides solubles et par la souche mutante 39EA utilisant des pentoses, des hexoses et autres glucides résultant de la dégradation de la biomasse cellulosique, comprend, en outre, un solvant, avantageusement constitué par un alcool aliphatique inférieur pris, de préférence, dans le groupe qui comprend le méthanol et l'éthanol ou une cétone, qui joue un rôle de stimulant de la croissance des cellules de la souche mutante 39EA.

Egalement conformément à l'invention, le système de bioconversion comprenant un substrat cellulosique, au moins un microorganisme ou une enzyme apte à dégrader la cellulose ou l'hémicellulose en hexoses, pentoses et autres glucides solubles et la souche mutante 39EA utilisant les sucres résultant de la dégradation de la biomasse cellulosique, comprend, en outre, un co-substrat pris dans le groupe des aldéhydes et des cétones.

Un tel système de bioconversion comprenant, conformément à l'invention, un substrat cellulosique, un solvant stimulant tel que défini plus haut, l'acétone comme co-substrat, la souche mutante 39EA et un microorganisme apte à dégrader la cellulose ou l'hémicellulose en hexoses et pentoses solubles, permet la production d'isopropanol.

Selon une modalité avantageuse de cette disposition, l'alcool aliphatique inférieur ou la cétone utilisés comme solvant, est présent à une concentration comprise entre 0,1 et 8,2 % en volume, et de préférence, de 0,1 à 6% environ dans le cas de l'acétone.

Selon une autre modalité avantageuse de cette disposition, le co-substrat est présent à une concentration de l'ordre de 1 à 6 % environ en volume.

Conformément à l'invention, le produit final obtenu par bioconversion peut être récupéré à la température à laquelle il est produit, qui est, de préférence, comprise entre 40 et 75°C, par distillation dudit produit, qui est volatil, en continu, sous pression réduite.

Egalement conformément à l'invention, la souche mutante 39EA et/ou les microorganismes exerçant une activité cellulolytique est ou sont mis en oeuvre en culture continue ou discontinue ou sous forme de cellules immobilisées.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de mise en oeuvre du procédé objet de la présente invention.

Il doit être bien entendu, toutefois, que ces exemples de mise en oeuvre sont donnés uniquement à titre d'illustration de l'objet de l'invention, dont ils ne constituent en aucune manière une limitation.

EXEMPLE 1 - Production d'éthanol en l'absence de solvants dans le système de bioconversion

1. Production de la souche mutante 39EA de C. thermohydrosulfuricum.

La souche sauvage 39E de C. thermohydrosulfuricum est peu résistante à l'éthanol et il a été établi que sa croissance est fortement inhibée lorsque la concentration d'éthanol dépasse 2 % en volume.

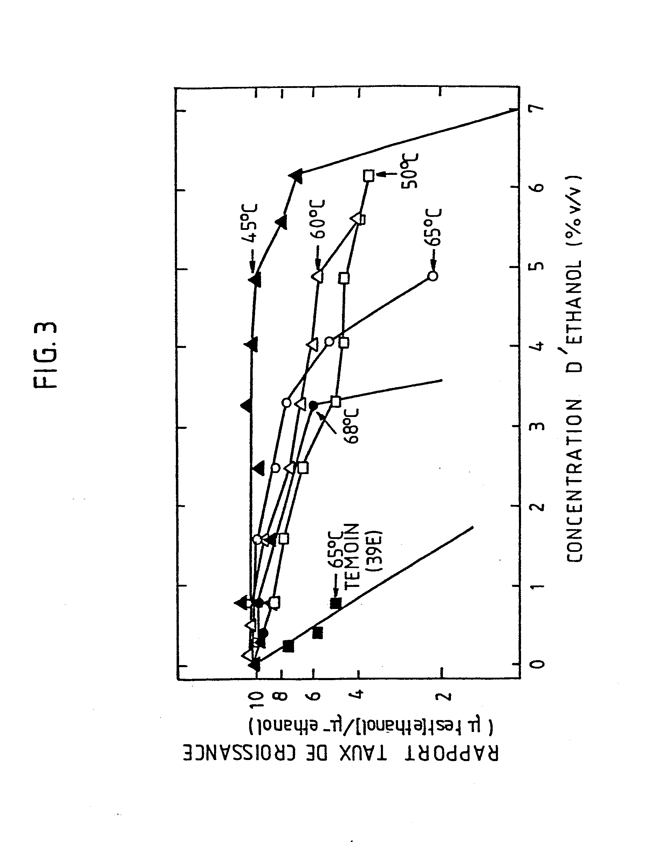

La souche mutante 39EA a été améliorée génétiquement pour être rendue résistante à l'éthanol. Elle a été obtenue par transferts successifs de la souche sauvage 39E dans des milieux de culture contenant des concentrations croissantes d'éthanol et elle a été ensuite isolée sur milieu solide contenant 6 % d'éthanol. Ses caractéristiques de croissance, comparées à celles de la souche sauvage 39E ont été réunies dans les tableaux I et II ci-dessus et dans la figure 3.

2. Production d'éthanol

La souche mutante 39EA pousse sur les mêmes milieux de culture que la souche sauvage 39E et notamment sur un milieu CM3-3 dont la composition, en g par litre, est la suivante :

$KH_2PO_4$ : 1,5 ; $K_2HPO_4$ : 2,2 ; $(NH_4)_2SO_4$ : 1,3 ;

$MgCl_2$, $6H_2O$ : 0,1 ; $CaCl_2.2H_2O$ : 0,01 ; $NaHCO_3$ : 5 ;

$FeSO_4.7H_2O$ : 0,0025 ; cystéine - HCl ou sulfure de sodium : 0,5 ;

Extrait de levure (Difco) : 5 ;

Sources de carbone (habituellement xylose ou glucose) : 5, stérilisées à 110°C pendant 30 minutes avant d'être introduites dans le milieu ;

1 ml d'une solution à 0,2 % de resazurine.

La préparation du milieu se fait sous flux d'un mé-

lange $N_2-CO_2$ (95 % - 5 %).

Le pH initial du milieu complet est égal à 7,4-7,5.

Alors que le rendement maximum d'éthanol obtenu par bioconversion de glucose par la souche sauvage 39E est de 1,6 à 1,8 (éthanol/glucose : mole/mole) et qu'il diminue considérablement lorsqu'on ajoute une faible proportion d'éthanol, contrairement à cela, le meilleur rendement obtenu à ce jour avec la souche mutante 39EA (éthanol/glucose : mole/mole) est sensiblement constant jusqu'à 4 % environ d'éthanol (% poids-/volume), avec un maximum à 2 %, ainsi que cela ressort des tableaux III à V qui vont suivre.

## TABLEAU III

Influence de la concentration initiale d'éthanol sur le bilan fermentaire
de la fermentation du glucose par la souche mutante 39 EA
de Clostridium thermohydrosulfuricum tolérante aux solvants.

| Ethanol (% Poids/Vol.) | Croissance (DO à 660 nm) | pH | Glucose consommé µmole/10ml | Formation de produits (µmoles/10 ml) | | | | Carbone récupéré (%) |
|---|---|---|---|---|---|---|---|---|
| | | | | Lactate | Ethanol | Acétate | $CO_2$ | |
| 0 | 0,75 | 5,0 | 164 | 130 | 150 | 17 | 154 | 92 |
| 0,8 | 0,70 | 5,1 | 155 | 132 | 135 | 13 | 104 | 94 |
| 2,0 | 0,76 | 5,0 | 211 | 137 | 217 | 15 | 233 | 90 |
| 4,0 | 0,60 | 5,0 | 147 | 129 | 116 | 14 | 131 | 87 |

-13-

0139546

Les résultats réunis dans le tableau III ci-dessus ont été obtenus dans les conditions suivantes : on a introduit dans des tubes résistants à la pression, pour cultures anaérobies, 10 ml de milieu TYE (contenant en g/l) : $NH_4Cl$ : 1,0 ; $KH_2PO_4$ : 0,3 ; $Na_2HPO_4$, $7H_2O$ : 2,1 ; $MgCl_2$, $6H_2O$ : 0,2 ; Bactotryptone : 10 ; Yeast Extract : 3,0 ; $FeSO_4$ : 0,000625 ; 5 ml d'une solution de vitamines contenant en g/l : Biotine : 0,002 ; acide folique : 0,002 ; pyridoxine-HCl : 0,001 ; thiamine, HCl : 0,005 ; riboflavine: 0,005 ; acide nicotinique : 0,005 ; acide pantothénique : 0,005 ; vitamine $B_{12}$ (cyanocobalamine) cristalline : 0,0001 ; acide para-aminobenzoïque : 0,005 ; acide lipoïque : 0,005 ; 10 ml d'une solution d'élements minéraux contenant en g/l acide nitrilotriacétique : 12,8 ; $FeSO_4$, $7H_2O$ : 0,1 ; $MnCl_2$, $4H_2O$ : 0,1 ; $CoCl_2$, $6H_2O$ : 0,17 ; $CaCl_2$, $2H_2O$ : 0,1 ; $ZnCl_2$ : 0,1 ; $CuCl_2$ : 0,02 ; $H_3BO_3$ : 0,01 ; Na Molybdate : 0,01 ; NaCl : 1,0 ; $Na_2SeO_3$ : 0,017 ; $NiSO_4$, $6H_2O$ : 0,026 ; 1 ml d'une solution à 0,2 % de résazurine.

Le pH est amené à 7,2-7,4. Après ébullition, on refroidit et dispense 9,6 ml de milieu par tube de culture anaérobie sous flux $N_2-CO_2$ (95 - 5). On autoclave 30 minutes à 120°C. On ajoute au moment de l'emploi 0,25 ml de $Na_2S$, $9H_2O$ à 25 g/l. Le milieu est supplémenté avec 220 μM de glucose, 2 μCurie de $^{14}C$-UL glucose.

Après inoculation de la souche mutante 39EA, les tubes ont été incubés pendant 36 heures à 63°C, sans agitation. La formation des produits a été déterminée par analyse de la radioactivité dans les produits finaux, après séparation par chromatographie en phase liquide sous haute pression (HPLC). Les résultats qui figurent dans le tableau III représentent la moyenne de deux expériences.

## TABLEAU IV

Influence de la concentration initiale d'éthanol sur le bilan des produits de la fermentation du glucose par la souche mutante 39EA de C. thermohydrosulfuricum.

| Produit (en mM 100 mM glucose consommé) / Ethanol (en %, poids/vol) | 0 | 0,8 | 2,0 | 4,0 |
|---|---|---|---|---|
| Lactate | 80 | 86 | 66 | 82 |
| Acétate | 10,4 | 9,0 | 7,2 | 9,5 |
| Anhydride carbonique | 99 | 93 | 111 | 94 |
| Ethanol | 92 | 84 | 99 | 74 |
| Rapport Ethanol/glucose (mole/mole) | 0,92 | 0,84 | 0,99 | 0,74 |

Nota :

1) Les résultats ci-dessus ont été calculés à partir des résultats indiqués dans le tableau III.

2) Avec la souche sauvage 39E, on n'observe pas de croissance en présence de 2,0 % d'éthanol (vol/vol) ; pour une addition initiale d'éthanol égale à 0 %, le rapport éthanol/glucose est de 1,6 (mole/mole) et pour une addition de 1,6 % d'éthanol, ce rapport devient égal à 0,63.

EXEMPLE 2 - Production d'éthanol en présence de solvants dans le système de bioconversion

La souche mutante 39EA, de même d'ailleurs que la souche sauvage 39E, sont plus ou moins affectées dans leur croissance et leur production de métabolites, en particulier leur production d'éthanol, lorsqu'elles sont cultivées dans des systèmes de bioconversion contenant des concentrations plus ou moins élevées de différents solvants.

Le tableau V ci-après montre l'influence qu'exercent différents solvants (donnés à titre d'exemples non limitatifs), présents à différentes concentrations, sur la crois-

0139546

-16-

sance de la souche 39E et de la souche 39EA, respectivement. Les données réunies dans ce tableau ont été obtenues par incubation desdites souches à 62°C, pendant 24 heures, sans agitation, sur un milieu CM3-3 contenant 5 g/ litre de xylose et 5 g/litre d'extrait de levure.

Dans les cas d'isopropanol (5% d'isopropanol dans le milieu de culture), on a noté une croissance pour les deux souches après 72 heures d'incubation (la croissance de la souche 39EA a été meilleure que celle de la souche 39E /le Tableau V/ , mais la souche 39EA a subi une lyse pendant que la mesure a été effectuée).

Toutefois, la souche mutante 39EA a une croissance bien meilleure que la souche sauvage 39E, tout au moins jusqu'aux concentrations en isopropanol de 2%.

## TABLEAU V

Comparaison de l'influence exercée par différentes concentrations

de solvants sur la croissance des souches 39E et 39EA

de C. thermohydrosulfuricum

| Sou-che | DO à 540 nm | SOLVANTS % (v/v) | | | | | | | | | | | | | | | | | | | | | |
|---------|---|---------|------|------|------|-------|------|------|------|------|-------------|------|------|------|---------|-----|------|---|--------|------|------|------|------|------|
| | | METHANOL | | | | ETHANOL | | | | | ISOPROPANOL | | | | BUTANOL | | | | ACETONE | | | | | |
| | | 0 | 1 | 2 | 5 | 0 | 1 | 2 | 4 | 6 | 0 | 1 | 2 | 5 | 0 | 1 | 2 | 5 | 0 | 1 | 2 | 4 | 5 | 6 |
| 39E | | 0,41 | 0,31 | 0,21 | 0 | 0,9 | 0 | 0 | 0 | 0 | 0,58 | 0,76 | 0,25 | 0,22 | 1,1 | 0,1 | 0 | 0 | 0,71 | 0,72 | 0,74 | 0 | 0 | 0 |
| 39EA | | 0,73 | 0,82 | 0,79 | 0,73 | 0,82 | 0,88 | 0,88 | 0,76 | 0,05 | 0,9 | 0,95 | 0,85 | 0,09 | 0,9 | 1,0 | 0,13 | 0 | 0,9 | 1,12 | 1,22 | 1,15 | 1,05 | 0,00 |

0139546

-18-

Il ressort de ce tableau qu'alors que la croissance de la souche 39E est totalement inhibée en présence de méthanol ou d'acétone présents à une concentration de 5 %, en présence d'éthanol présent à une concentration de 4 %, en présence d'isopropanol présent à une concentration de 2 %, et en présence de butanol à une concentration de 1 %, la croissance de la souche 39EA n'est pas inhibée aux mêmes concentrations de ces solvants. De plus, la croissance de 39EA est stimulée par l'addition des alcools et cétone testés.

Il y a lieu de mentionner que l'addition de solvant dans le milieu de bioconversion peut être réalisée indifféremment soit au début, soit à la fin de la fermentation.

Il peut être avantageux de réaliser l'opération sous vide partiel, car dans de telles conditions, le solvant ajouté peut jouer un rôle d'entraînement du composé final produit, facilitant ainsi la récupération de ce dernier en provoquant la formation d'un azéotrope ternaire, par exemple.

EXEMPLE 3 - Production d'éthanol en continu en présence d'une concentration élevée de méthanol dans le système de bioconversion

L'addition de 5 % de méthanol (V/V) dans le système de bioconversion se prête à une culture continue de C. thermohydrosulfuricum 39EA et à une production résultante continue d'éthanol. Dans ces conditions, le méthanol peut servir, en fin d'opération, d'entraînement pour la récupération de l'éthanol produit, par formation d'un azéotrope ternaire qui permet la distillation, sous vide partiel, de l'éthanol produit ; en effet, la croissance de la souche 39EA ayant lieu à des températures élevées (de l'ordre de 62°C, par exemple), de telles températures sont propices à la récupération en continu de l'éthanol produit.

Le tableau VI ci-après montre la production d'éthanol dans une culture continue de 39EA en présence de 5 % de méthanol (v/v). La croissance de 39EA est réalisée sur un milieu CM3-3 contenant 5 g/litre d'extrait de levure et 5 % de méthanol (v/v), avec diverses concentrations de xylose, à une température de 62°C, un pH de 7,0, sous agitation à 100 tours/minute, dans un fermenteur d'un volume de 350 ml. On maintient un flux de $CO_2$ de l'ordre de 5 ml/minute.

TABLEAU VI

Production continue d'éthanol par fermentation de xylose dans une culture continue de C. thermohydrosulfuricum 39EA en présence de 5 % de méthanol (v/v).

| Concentration de xylose dans le milieu d'entrée (g/litre) | Xylose résiduel dans le fermenteur (g/litre) | Taux de dilution D ($h^{-1}$) | DO à 540 nm | Ethanol produit (mM) |
|---|---|---|---|---|
| 2 | non mesuré 0,68 | 0,19 0,26 | 0,54 0,41 | 13,5 |
| 5 | 3,2 | 0,19 | 0,9 | 11,5 |
| 10 | 5,9 | 0,19 | 2,2 | 11,1 |
| 20 | 15,35 | 0,19 | 2,15 | 12,4 |

L'éthanol peut être récupéré en phase vapeur à sa température de production (60 à environ 75°C) et est continuellement entraîné de la phase liquide dans la phase vapeur en faisant passer un gaz inerte dans le fermenteur.

EXEMPLE 4 - Production d'isopropanol en présence d'un co-substrat constitué par l'acétone

On trouvera dans le tableau VII qui va suivre les résultats obtenus respectivement avec un système de bioconversion comprenant la souche sauvage 39E et avec un système de bioconversion comprenant la souche mutante 39EA.

Les opérations respectives ont été menées en discontinu, en inoculant la souche testée sur un milieu CM3-3 contenant 5 g/litre de xylose, 5 g/litre d'extrait de levure et 1 % ou 5 % (vol/vol) d'acétone et en incubant pendant 24

-20-

heures à 62°C, sans agitation.

TABLEAU VII

Comparaison de la production d'isopropanol par fermentation
de xylose respectivement par les souches 39E et 39EA
en présence d'acétone.

| Souche | Crois-sance (DO à 540 nm) | Concentration en acétone % vol/vol | pH | Produits finaux (mM) | | |
|---|---|---|---|---|---|---|
| | | | | Iso-pro-panol | Acé-tate | Lac-tate |
| 39E | 0,9 | 1 | 6,1 | 63 | 37 | 6 |
| 39EA | 1,2 | 1 | 6,0 | 69 | 32 | 6 |
| 39E | 0 | 5 | - | - | - | - |
| 39EA | 1,05 | 5 | | 125 | | |

EXEMPLE 5 - Production d'isopropanol en présence d'un
co-substrat et d'un solvant.

La souche mutante 39EA de C. thermohydrosulfuricum
est mise en culture continue sur un milieu de culture CM3-3
contenant différentes concentrations de xylose, 5 g/litre
d'extrait de levure, 1 % d'acétone (vol/vol) comme co-
substrat et différentes concentrations d'éthanol comme solvant, à une température de 62°C, à un pH de 7,0, sous agitation à 100 tours/minute, dans un fermenteur d'un volume de
350 ml. Les résultats obtenus sont réunis dans le tableau
VIII ci-après.

-21-

TABLEAU VIII

Production continue d'isopropanol par fermentation de xylose
dans une culture continue de C. thermohydrosulfuricum 39EA
en présence de fortes concentrations d'éthanol
et de 1 % d'acétone (v/v)

| Concentration d'éthanol dans le milieu d'entrée (% vol/vol) | Concentration de xylose dans le milieu d'entrée (g/litre) | $D(h^{-1})$ | DO (540nm) | Concentration d'iso-propanol produit (mM) |
|---|---|---|---|---|
| 3,3 | 2 | 0,09 | 1,3 | 33 |
| | 2 | 0,2 | 1,27 | 46 |
| | 2 | 0,44 | 1,24 | 45 |
| 5,5 | 5 | 0,13 | 1,38 | 66 |
| 7,2 | 5 | 0,07 | 1,45 | 76 |

EXEMPLE 6 - Production de métabolites présentant un intérêt
industriel par fermentation de la cellulose par
des co-cultures de souche mutante 39EA et de souches cellulolytiques.

Ainsi qu'on l'a mentionné plus haut, on sait que la
fermentation de la cellulose par une co-culture de Clostridium thermocellum LQRI et de Clostridium thermohydrosulfuricum 39E ne permet pas d'obtenir des concentrations d'éthanol
supérieures à 1 %, la croissance de C. thermocellum étant,
par ailleurs, inhibée en présence de concentrations d'éthanol
d'environ 1 %.

On a introduit dans des tubes pour cultures anaérobies, 10 ml de milieu CM3-3, 1 % d'acétone, 1 % de méthanol
(v/v) et 0,2 g de cellulose (de marque "SOLKA FLOC").

-22-

Une première série de tubes a été inoculée par 1 ml de culture de 39EA ayant poussé sur milieu TYE contenant 5 g/litre de glucose et 5 % d'éthanol (v/v).

Une deuxième série de tubes a été inoculée par 1 ml de culture de LQRI ayant poussé sur milieu CM3-3.

Une troisième série de tubes a été inoculée à la fois par 1 ml de culture de 39EA et par 1 ml de culture de LQRI.

Les trois séries de tubes ont été incubées pendant 5 jours à 60°C, sans agitation.

Les résultats obtenus sont réunis dans le tableau IX ci-après :

TABLEAU IX

Fermentation de la cellulose respectivement par des monocultures et par des co-cultures de C. thermohydrosulfuricum souche 39EA et de C. thermocellum souche LQRI, en présence de concentrations élevées de solvants.

| Conditions | Produits de fermentation (mM) | | |
|---|---|---|---|
| | Ethanol | Acétate | Isopropanol |
| Pas d'inoculation | 5 | < 5 | 5 |
| 39EA | 41 | < 5 | 48 |
| LQRI | 5 | 31 | 63 |
| 39EA + LQRI | 43 | 41 | 85 |

Il ressort de ce tableau que dans ce milieu contenant des concentrations élevées de solvants (1 % de méthanol et 1 % d'acétone) : (i) la souche LQRI pousse sur cellulose (croissance mise en évidence par la formation d'acétate) et convertit une partie de l'acétone en isopropanol et (ii) dans le cas de la co-culture de 39EA avec LQRI, la croissance est plus importante (production supérieure d'acétate) et, de plus, la production d'isopropanol est plus élevée ; cela montre que les dextrines ou hexoses obtenus par dégradation de la cellulose par C. thermocellum sont utilisés par la souche 39EA pour la croissance (production d'acétate) et pour la production d'isopropanol.

Les composés produits par bioconversion conformément à la présente invention peuvent être récupérés assez facilement avec une consommation d'énergie faible, et sont obtenus à des concentrations supérieures à celles que permettent d'atteindre les procédés proposés dans l'Art antérieur, donc avec des rendements de production nettement améliorés.

De plus, la possibilité de réaliser la bioconversion à l'aide de cultures continues du microorganisme permet une réduction des investissements en appareillages.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

REVENDICATIONS

1°) Procédé de production d'alcools par bioconversion d'au moins un substrat constitué par des composés organiques oxygénés, à l'aide d'au moins un microorganisme thermophile anaérobie résistant aux alcools obtenus, à température élevée, de préférence comprise entre 40 et 75°C, et plus particulièrement à la température de croissance du ou des microorganisme(s) thermophile(s) anaérobie(s), en présence de concentrations égales ou supérieures à 1 % dudit produit final.

2°) Procédé selon la revendication 1, caractérisé en ce que ledit substrat est soumis à l'action de bioconversion d'au moins un microorganisme thermophile anaérobie, en présence d'un solvant organique liquide de préférence constitué par un alcool aliphatique inférieur ou une cétone, qui stimule la croissance et/ou la production de l'alcool obtenu en tant que produit final.

3°) Procédé selon la revendication 1, caractérisé en ce que ledit substrat est associé à un co-substrat constitué par un solvant pris dans le groupe des cétones et des aldéhydes.

4°) Procédé selon la revendication 3, caractérisé en ce que le substrat et le co-substrat sont associés à un solvant organique liquide de préférence constitué par un alcool aliphatique inférieur ou une cétone.

5°) Procédé selon la revendication 1, caractérisé en ce que ledit substrat est constitué par au moins un saccharide pris dans le groupe qui comprend les pentoses, les hexoses, et autres glucides, qui est soumis à l'action de bioconversion d'au moins un microorganisme thermophile anaérobie résistant au produit organique réduit obtenu, pour fournir un alcool aliphatique inférieur à une concentration égale ou supérieure à 1 % et pouvant atteindre au moins 5 % (v/v) dans le système de bioconversion.

6°) Procédé selon la Revendication 5, caractérisé en ce que l'alcool aliphatique inférieur obtenu est de l'éthanol.

- 25 -

7°) Procédé selon les Revendications 5 et 6, caractérisé en ce que le système de bioconversion comprend en outre un solvant constitué par un alcool aliphatique inférieur ou une cétone, qui joue le rôle de stimulant de la croissance du microorganisme, et/ou de la production de l'alcool aliphatique inférieur recherché en tant que produit final.

8°) Procédé selon les Revendications 5 et 6, caractérisé en ce que le système de bioconversion comprend en outre un co-substrat constitué par un solvant pris dans le groupe des cétones et des aldéhydes.

9°) Procédé selon la Revendication 8, caractérisé en ce que le système de bioconversion comprend en outre un solvant constitué par un alcool aliphatique ou une cétone, qui stimule la bioconversion du co-substrat mis en oeuvre.

10°) Procédé selon la Revendication 7 ou la Revendication 9, caractérisé en ce que l'alcool aliphatique inférieur ou la cétone est pris dans le groupe qui comprend le méthanol, l'éthanol, l'isopropanol, le butanol et l'acétone.

11°) Procédé selon la Revendication 10, caractérisé en ce que l'alcool ou la cétone est présent dans le système de bioconversion à une concentration comprise entre 0,1 et 8,2 % en volume.

12°) Procédé selon la Revendication 11, caractérisé en ce que dans le cas où l'éthanol est utilisé comme stimulant, sa concentration dans le système de bioconversion est comprise entre 0,1 et 5% en volume.

13°) Procédé selon la Revendication 11, caractérisé en ce que dans le cas où le méthanol est utilisé comme stimulant, sa concentration dans le système de bioconversion est comprise entre 0,1 et 8,2% en volume.

14°) Procédé selon la Revendication 8, caractérisé en ce que dans le cas où l'acétone est utilisé comme co-substrat, sa concentration dans le système de bioconversion est comprise entre 0,1 et 6% en volume.

-26-

15°) Procédé selon la Revendication 3, caractérisé en ce que le système de bioconversion comprend un substrat constitué par au moins un saccharide, un microorganisme thermophile anaérobie, un co-substrat pris dans le groupe qui comprend les aldéhydes et les cétones, et qui est avantageusement constitué par de l'acétone, et le produit final obtenu est essentiellement de l'isopropanol.

16°) Procédé selon la Revendication 4, caractérisé en ce que le système de bioconversion comprend un substrat constitué par au moins un saccharide, un microorganisme thermophile anaérobie, un co-substrat pris dans le groupe qui comprend les aldéhydes et les cétones, et est avantageusement constitué par de l'acétone, et un solvant organique liquide avantageusement constitué par un alcool aliphatique inférieur pris, de préférence, dans le groupe qui comprend le méthanol, l'éthanol et le butanol, le produit final obtenu par bioconversion étant essentiellement de l'isopropanol.

17°) Procédé selon la Revendication 15, caractérisé en ce que le co-substrat avantageusement constitué par de l'acétone est présent à une concentration de 0,1 à 6% en volume dans le système de bioconversion, et l'isopropanol est produit à des concentrations comprises entre 0,1 et plus de 2% (v/v).

18°) Procédé selon la Revendication 17, caractérisé en ce que l'isopropanol est produit à une concentration d'environ 6 % (v/v).

19°) Procédé selon la Revendication 16, caractérisé en ce que le co-substrat tel que l'acétone est présent à une concentration de 0,1 à 6% en volume dans le système de bioconversion, et l'alcool aliphatique inférieur est présent à une concentration comprise entre 0,1 et 8,2% en volume, pour produire de l'isopropanol à une concentration comprise entre 0,1 et plus de 2% (v/v).

20°) Procédé selon la Revendication 19, caractérisé en ce que l'isopropanol est produit à une concentration d'environ 6% (v/v).

- 27 -

21°) Procédé selon l'une quelconque des revendications 1 à 20, caractérisé en ce que le microorganisme thermophile anaérobie mis en oeuvre dans le système de bioconversion est de préférence constitué par une souche mutante d'une bactérie thermophile anaérobie telle que Clostridium thermosaccharolyticum ou Clostridium thermohydrosulfuricum, obtenue après transferts successifs de la souche sauvage de ladite bactérie thermophile dans des milieux de culture contenant des concentrations croissantes d'éthanol et isolément sur milieu solide contenant 6% d'éthanol.

22°) Procédé selon la Revendication 21, caractérisé en ce que la souche mutante de Clostridium thermohydrosulfuricum est la souche 39EA, déposée dans la Collection Nationale de Cultures de Microorganismes tenue par l'INSTITUT PASTEUR, sous le n°I-226 en date du 16 JUIN 1983, obtenue après transferts successifs de la souche sauvage de Clostridium thermohydrosulfuricum 39E dans des milieux de culture contenant des concentrations croissantes d'éthanol, et isolément sur milieu solide contenant 6% d'éthanol.

23°) Procédé selon la Revendication 21 et/ou la Revendication 22, caractérisé en ce que la souche mutante 39EA est associée à des microorganismes ou des enzymes aptes à dégrader d'autres saccharides que les pentoses, les hexoses et autres glucides, et notamment à des microorganismes ou des enzymes aptes à dégrader la cellulose et l'hémicellulose en sucres solubles.

24°) Procédé selon la Revendication 23, caractérisé en ce que le substrat est constitué par de la cellulose ou de l'hémicellulose.

25°) Procédé selon la Revendication 23, caractérisé en ce que les microorganismes aptes à dégrader la cellulose et l'hémicellulose en hexoses, pentoses et autres glucides solubles sont des microorganismes exerçant une activité cellulolytique tels que, notamment, Clostridium thermocellum souche LQRI et les enzymes cellulolytiques sont de préférence des complexes cellulasiques ou hémicellulasiques pour

- 28 -

pour produire un alcool aliphatique inférieur comme l'éthanol à une concentration égale ou supérieure à 1% dans le système de bioconversion, jusqu'à au moins 5% (v/v).

26°) Procédé selon la Revendication 23, caractérisé en ce que le système de bioconversion comprenant un substrat du type de la cellulose ou de l'hémicellulose, au moins un microorganisme ou une enzyme apte à dégrader l'hémicellulose et la cellulose en hexoses, pentoses et autres glucides solubles et la souche mutante 39EA utilisant les hexoses, les pentoses et autres glucides résultant de la dégradation de la biomasse cellulosique, comprend, en outre, un solvant, avantageusement constitué par un alcool aliphatique inférieur pris, de préférence, dans le groupe qui comprend le méthanol et l'éthanol, qui joue un rôle de stimulant de la croissance des cellules de la souche mutante 39EA.

27°) Procédé selon la Revendication 23, caractérisé en ce que le sytème de bioconversion comprenant un substrat cellulosique, au moins un microorganisme ou une enzyme apte à dégrader la cellulose ou l'hémicellulose en hexoses, pentoses et autres glucides solubles et la souche mutante 39EA, comprend, en outre, un co-substrat pris dans le groupe des aldéhydes et des cétones.

28°) Procédé selon la Revendication 23, caractérisé en ce que le système de bioconversion comprenant un substrat cellulosique, au moins un microorganisme ou une enzyme apte à dégrader l'hémicellulose et la cellulose en pentoses, hexoses et autres glucides solubles et la souche mutante 39EA de bioconversion des pentoses, des hexoses et autres glucides résultant de la dégradation de la cellulose ou de l'hémicellulose, comprend, en outre, un co-substrat pris dans le groupe des aldéhydes et des cétones et un solvant avantageusement constitué par un alcool aliphatique inférieur pris, de préférence, dans le groupe qui comprend le méthanol et l'éthanol, ou le butanol, l'alcool aliphatique inférieur obtenu en tant que produit final étant, dans le cas où l'acétone est le co-substrat, essentiellement de l'isopropanol.

29°) Procédé selon la Revendication 27, caractérisé en ce que le co-substrat est présent à une concentration del'ordre de 0,1 à 6% environ en volume.

30°) Procédé selon la Revendication 26, ou la Revendication 28, caractérisé en ce que l'alcool aliphatique inférieur ou la cétone utilisés comme solvant, sont présents à une concentration comprise entre 0,1 et 8,2% en volume, et de 0,1 à 6% dans le cas de l'acétone.

31°) Procédé selon l'une quelconque des Revendications 23 à 30, caractérisé en ce que dans le cas où on utilise pour dégrader la cellulose ou l'hémicellulose des enzymes de préférence constituées par des complexes cellulasiques ou hémicellulasiques, ceux-ci sont récupérés, une fois la bioconversion réalisée, pour être recyclés dans une nouvelle charge de bioconversion.

32°) Procédé selon l'une quelconque des Revendications 1 à 31, caractérisé en ce que le produit final obtenu par bioconversion est récupéré à la température à laquelle il est produit, qui est, de préférence, comprise entre 40 et 75°C, par distillation dudit produit, qui est volatil, en discontinu ou continu, sous pression réduite et éventuellement avec fluage de gaz inerte à travers le fermenteur.

33°) Procédé selon l'une quelconque des Revendications 1 à 32, caractérisé en ce que le ou les microorganisme(s) mis en oeuvre dans la bioconversion est ou sont en culture continue ou discontinue ou sous forme de cellules immobilisées ou dans un système équivalent.

34°) Souche mutante de Clostridium thermohydrosulfuricum, dénommée souche 39EA, déposée dans la Collection Nationale de Cultures de Microorganismes tenue par l'INSTITUT PASTEUR, sous le n°I-226, en date du 16 JUIN 1983, obtenue après transferts successifs de la souche sauvage de C.thermohydrosulfuricum 39E dans des milieux de culture contenant des concentrations croissantes d'éthanol et isolément sur milieu solide contenant 6% d'éthanol.

35°) Alcool aliphatique inférieur produit par la souche 39EA ou une souche équivalente ou dérivée, mettant en oeuvre une ou plusieurs étapes du procédé selon l'une quelconque des Revendications 1 à 33.

36°) Souche mutante 39EA selon la Revendication 34, capable de produire de l'éthanol ou de l'isopropanol à une concentration supérieure à 1% à partir d'hexoses ou de pentoses ou autres glucides.

37°) Souche mutante selon la Revendication 34, capable de produire de l'éthanol ou de l'isopropanol à une concentration supérieure à 4% à partir d'hexoses ou de pentoses ou d'autres glucides.

38°) Souche mutante 39EA selon la Revendication 34, capable de produire de l'éthanol ou de l'isopropanol à une concentration supérieure à 1% en utilisant des hexoses, des pentoses ou d'autres glucides obtenus par hydrolyse de biomasse cellulosique.

39°) Procédé selon les Revendications 18 et 20, caractérisé en ce que l'isopropanol présente une pureté optique élevée.

FIG. 1

0139546

# FIG. 2

FIG. 3